# EUROPÄISCHE PATENTSCHRIFT

(11) **EP 1 725 180 B1**
(45) Veröffentlichungstag und Bekanntmachung des Hinweises auf die Patenterteilung: **25.08.2010**
(21) Anmeldenummer: 04736428.6
(22) Anmeldetag: 09.06.2004
(51) Int. Cl.: A61B 19/02

(54) **BEHÄLTER**
CONTAINER
RECIPIENT

(30) Priorität: 07.03.2004 CH 3932004
(43) Veröffentlichungstag der Anmeldung: 29.11.2006
(73) Patentinhaber: Synthes GmbH, 4436 Oberdorf (CH)
(72) Erfinder: CANDRIAN, Karina, CH-8700 Küsnacht (CH); FORRER, Ruth, CH-4313 Möhlin (CH); FURRER, André, CH-4571 Lüterkofen (CH)
(74) Vertreter: Rosenich, Paul
(86) Internationale Anmeldenummer: PCT/IB2004/001882
(87) Internationale Veröffentlichungsnummer: WO 2005/092231

(56) Entgegenhaltungen:
- DE-A- 4 122 045
- DE-U- 29 705 944
- US-A- 5 881 878
- US-A1- 2001 042 696

## Beschreibung

Die Erfindung betrifft einen Behälter und ein Trägersystem nach dem Oberbegriff der unabhängigen Ansprüche für chirurgisches Hilfsmaterial.

Aus dem Stand der Technik sind Aufbewahrungs-, Transport- und Darreichungssysteme von chirurgischen Hilfsmaterialien, wie Schrauben, Nägel und Stifte, bekannt, die zusammen mit Implantaten einem Chirurgen bei einer Operation zur Verfügung stehen.

Die Implantate und die Hilfsmaterialien für die Befestigung der Implantate im und am Patienten müssen sterilisiert werden und sollten möglichst lange, steril aufbewahrt werden können. In ihrer Aufbewahrungslage bzw. in ihren Behältern sollten die Hilfsmaterialien auch gewaschen werden können, ohne, dass man sie dazu extra aus dem Behälter nehmen muss.

Aus der Patentliteratur ist durch DE-A1-41 22 045 bekannt, dass Knochenschrauben in Sterilisationsbehältern aufbewahrt werden können. In diesem Zusammenhang hat DE-A1-41 22 045 vorgeschlagen, eine Lochplatte zu verwenden, auf der mehrere Behälter stabil positioniert werden. Ein solcher Behälter bietet Platz für zehn Knochenschrauben.

Am Fachmarkt erhältlich ist ein Angebot eines Unternehmens, das unter KLS-Martin, mit Sitz in Florida, USA, firmiert. Im Angebot sind Tabletts mit unterschiedlichen Abteilen für die Aufnahme von chirurgischen Instrumenten und Werkzeugen. Eine Lochplatte, die auch aus der DE-A1-41 22 045 bekannt ist, befindet sich in einem der Abteile. In der Lochplatte steckt unmittelbar eine grosse Anzahl an Schrauben, insbesondere Knochenschrauben. Die Schrauben können alle gleichzeitig aufgenommen werden. Wenn einmal ein solches Tablett im Krankenhausalltag verrutscht, oder in Kopflage gerät, können die Schrauben auf den Boden fallen.

Von dem gleichen Anbieter wird darüber hinaus auch ein Kunststoffsystem angeboten, das ebenfalls ein sterilisierbares Tablett darstellt. Das Schraubenabteil besteht aus einer Vielzahl länglicher, ovaler Steckplätze, in die Schraubenhalterungen bzw. Träger für Schrauben gesteckt werden können. Jede Schraubenaufnahme bzw. Träger ist mit einem Deckel versehen, unter dem sich im befüllten Zustand eine Gruppe von gleichartigen Schrauben, in der Regel von fünf gleichartigen Schrauben, befindet.

Ein weiteres Behältersystem für chirurgisches Hilfsmaterial ist aus WO9639091 bekannt.

Ein Chirurg oder sein Operationsteam stehen bei komplizierten Operationen häufig unter enormer psychischer Anspannung. Dabei soll jeder Handgriff präzise ausführbar sein. Besonders mit kleinen Hilfsmitteln, die für die Befestigung der Implantate benötigt werden, haben Chirurgen Probleme, da ihre Hände häufig grösser sind, wie zum Beispiel die Schrauben, die am Patienten eingesetzt werden sollen und welche die Implantate an den darunter liegenden Knochen befestigen sollen.

Der Chirurg möchte in der Regel immer nur jene Schraube mit seinem Werkzeug aufnehmen können, die er in dem jeweiligen Moment tatsächlich auch benötigt. Das gesamte restliche Hilfsmaterial soll so weit gesichert bleiben können, bis es benötigt wird. Es soll in dem gesicherten Zustand auch leichten Erschütterungen ausgesetzt sein dürfen, ohne ihre sterile Eigenschaft zu verlieren, indem es zum Beispiel auf den Boden fällt. Denn sobald etwas auf den Boden fällt, ist es auch im gesicherten Zustand in der Regel unsteril. Bevorzugt sollten also die Träger das Tablett nicht unabsichtlich verlassen dürfen.

In Vorbereitung auf eine Operation können entsprechende Trägersysteme zusammengestellt werden. Insbesondere bei Notoperationen ist für die Zusammenstellung nur eine sehr kurze Zeit bemessen. Im Sinne einer schnellen Zusammenstellung sollte ein Trägersystem leicht in den richtigen Mengen bestückbar sein, und es sollte nach dem Bestücken den rauen Krankenhausalltag überstehen können, ohne aus dem Tablett zu Boden fallen zu können. Gleichzeitig darf die dem obigen scheinbar konträre Anforderung nicht vernachlässigt werden, dass das Hilfsmaterial leicht zugänglich bzw. entnehmbar und während einer Vorbereitung gegebenenfalls erneut sterilisiert werden kann.

Diese und andere Aufgaben werden erfindungsgemäss durch den kennzeichnenden Teil eines Behälters nach Anspruch 1 gelöst. Vorteilhafte Ausgestaltungen sind in den abhängigen Ansprüchen zu finden.

Der Behälter weist einen Träger für die Lagerung und Darbietung von chirurgischem Hilfsmaterial, insbesondere Schrauben wie Knochenschrauben, in Aufnahme- bzw. Montagelage, und. zumindest eine abnehmbare Abdeckung zur Verlustsicherung des Hilfsmaterials auf. Das chirurgische Hilfsmaterial ist vereinzelt darbietbar. Erfindungsgemäss sind zumindest zwei derartige Behälter miteinander separierbar verbunden über die Abdeckung und/oder über den Träger.

Unter dem Begriff "vereinzelt darbietbar" wird hierin verstanden, dass das Hilfsmaterial dem Chirurgen einzeln angeboten werden kann. Dies steht im Gegensatz zu losem Hilfsmaterial, wie es beispielsweise in einer Schachtel enthalten ist. Darin sind die Schrauben unsortiert und zufällig verteilt enthalten. Vereinzelt darbietbare Schrauben wurden jedoch bereits vor dem Darbieten an den Chirurgen vereinzelt, d. h. aus dem losen Verband, in dem das Hilfsmaterial üblicherweise geliefert wird, gelöst und geordnet. Bevorzugt ist das Hilfsmaterial nicht nur vereinzelt darbietbar sondern sogar einzeln darbietbar. Dies bedeutet, dass in jedem Behälter nur ein einzelnes bzw. einziges Hilfsmaterial, d. h. nur ein Stück Hilfsmaterial pro Behälter, angeboten wird.

Ein Ausführungsbeispiel der Erfindung sieht vor, dass das chirurgische Hilfsmaterial dadurch vereinzelt darbietbar ist, dass der Träger nur eine Aufnahmeposition zur Aufnahme eines einzigen Hilfsmaterials aufweist. Eine andere Ausführung sieht vor, dass das Hilfsmaterial dadurch vereinzelt darbietbar ist, dass für jede einzelne Aufnahmeposition eine abnehmbare Abdeckung vorgesehen ist. Die vereinzelte Darbietbarkeit wird folglich zum einen dadurch gewährleistet, dass in jedem Träger eines Behälters nur ein einziges Hilfsmaterial aufgenommen werden kann. Hierfür weist jeder Träger nur eine einzige Aufnahme auf. Vereinzeltes und einzelnes Darbieten des Hilfsmaterials fallen in dieser Ausführungsform folglich zusammen. Zum anderen wird die vereinzelte Darbietbarkeit aber dadurch gewährleistet, dass jedes einzelne Stück Hilfsmaterial von einer einzigen Abdeckung abgedeckt wird. Hierbei können in einem Behälter also auch mehrere Hilfsmaterialien enthalten sein, natürlich vereinzelt angeboten. Der Träger kann hierzu mehrere Aufnahmepositionen aufweisen, in denen jeweils ein Hilfsmaterial aufgenommen werden kann. Diese werden von jeweils einer Abdeckung abgedeckt.

Unter dem Begriff "separierbar verbunden" wird hierin verstanden, dass die Behälter zunächst miteinander verbunden sind. Diese Verbindung kann jedoch vom Chirurgen gelöst werden. Die Behälter sind also separierbar. Eine Ausführung sieht hierbei vor, dass zumindest zwei Behälter über deren Abdeckungen separierbar verbunden sind. Gemäss einer weiteren Ausführung sind zumindest zwei Behälter über deren Träger separierbar verbunden. Bevorzugt erfolgt die Verbindung bereits bei der Herstellung der Behälter. Zum Beispiel können mehrere Träger oder mehrere Abdeckungen miteinander verbunden, d. h. in einem Stück hergestellt werden. Die Verbindung zwischen den Trägern bzw. den Abdeckungen ist jedoch vergleichsweise schwach, so dass einzelne Träger bzw. Abdeckungen abgebrochen werden können, wodurch sie separiert werden. Bevorzugt ist eine Sollbruchstelle vorgesehen. Gemäß einer anderen Variante erfolgt die separierbare Verbindung über einen Stöpsel auf dem einen Element und eine korrespondierende Öffnung auf dem anderen Element.

Eine weitere Ausführungsform eines Behälters weist wiederum einen Träger für die Lagerung und Darbietung von chirurgischem Hilfsmaterial, insbesondere Schrauben wie Knochenschrauben, in Aufnahme- bzw. Montagelage, und zumindest eine abnehmbare Abdeckung zur Verlustsicherung des Hilfsmaterials auf. Gemäss der Erfindung ist das chirurgische Hilfsmaterial vereinzelt darbietbar, dadurch, dass zumindest zwei Behälter für je ein einzelnes Hilfsmaterial miteinander separierbar verbunden sind über die Abdeckung und/oder über den Träger. Alternativ ist erfindungsgemäss das chirurgische Hilfsmaterial dadurch vereinzelt darbietbar, dass für jede einzelne Aufnahmeposition zur Aufnahme eines einzigen Hilfsmaterials in einem Behälter, in dem mehrere Hilfsmaterialien aufnehmbar sind, eine eigene abnehmbare Abdeckung vorgesehen ist, wobei die einzelnen Abdeckungen miteinander separierbar verbunden sind.

Ein Träger für chirurgisches Hilfsmaterial, wie Stifte, Nägel, aber vor allem Schrauben wie Knochenschrauben, hat erfindungsgemäße eine Lagerfläche und wenigstens ein Bein oder wenigstens zwei Beine. Der Träger hat die Form eines Tisches, dessen Lagerauflagefläche, das ist die nach oben gerichtete Oberfläche der Lagerfläche, dazu bestimmt ist, eine chirurgische Schraube oder ein Schraubenpaar o.dgl. zu tragen. Die Beine ragen im Wesentlichen im rechten Winkel aus der der Lagerauflagefläche gegenüberliegenden Seite heraus. Ein Träger ist so ausgestaltet, dass er nur ein vereinzeltes Hilfsmaterial, insbesondere eine vereinzelte Schraube oder ein vereinzeltes Schraubenpaar, in einer Halterung so aufnehmen kann, dass der Kopf des Hilfsmaterials bzw. der Kopf der Schraube auf der Lagerauflagefläche liegt und dessen Schaft zwischen den Beinen bzw. parallel zu den Beinen steckt.

Die Lagerfläche ist insbesondere mit wenigstens einer Rückbildung, die auch als Ausnehmung oder Aussparung bezeichnet werden kann, versehen, die so bemessen ist, dass ein passender Zahn der Abdeckung über die Rückbildung auf die der Lagerauflagefläche gegenüberliegende Seite gleiten kann. Dadurch erfolgt eine Verankerung der Abdeckung auf dem Träger.

Der Träger ist durch seine erfindungsgemässen Merkmale so gestaltet, dass er das chirurgische Hilfsmaterial günstig vereinzeln kann. Jedes einzelne Hilfsmaterial wird folglich gesondert in der entsprechenden einzelnen oder vereinzelbaren Aufnahme aufgenommen. Bevorzugt kann jeder Träger insgesamt nur eines (oder wenige, insbesondere zwei bzw. ein Paar) der Hilfsmaterialien aufnehmen und verwahren. Durch die Lage des Schwerpunkts und die Abschirmung der Beine und der Lagerfläche sitzt das Hilfsmaterial unter gewöhnlichen Umständen sicher in und auf dem Träger. Nahezu die gesamte Oberfläche des Hilfsmaterials ist frei zugänglich und kann so sterilisiert werden, ausser dort wo der Träger die Schraube umfasst. Auch hier wird die Schraube bzw. das Hilfsmaterial jedoch problemlos steril, weil es dort Spiel hat. In der Praxis werden die Träger mit den Schrauben immer wieder in einer Wasch-Spülmaschine gewaschen und anschließend sterilisiert.

Die erfindungsgemässen Rückbildungen, die an den Seiten des Trägers vorhanden sind, erfüllen zwei Funktionen. Zum einen können die Zähne der Abdeckung unter den Rückbildungen einrasten. Wenn der Träger zwischen Daumen und Zeigefinger genommen wird, erleichtern die Rückbildungen zum anderen das Ergreifen, des kleinen Gegenstandes. Doch im Krankenhausalltag wird in der Regel mit einer Pinzette gegriffen, so dass besondern vorteilhaft ist, Angriffsflächen für die Pinzette mittels Rückbildungen zu bieten.

Die Beine des Trägers münden in Kufen, die auch als Rastschienen bezeichnet werden können. Diese weisen insgesamt eine gerundete Form auf und deuten lateral nach Aussen weg. In einer alternativen Ausführungsform (je nach Ausbildung der Gleitschienen) können die Beine auch nach Innen zeigen. Die Kufen verbessern - bei Ausbildung von wenigstens zwei Beinen - das Stehvermögen des Trägers, wenn die Kufen länger als die Schrauben sind. In den Fällen, in denen die Träger kürzer als die Schrauben sind, übernehmen die Kufen eine andere Funktion zusammen mit dem Trägersystem. Werden die Träger mit einem Trägersystem verbunden, so rasten die Träger erfindungsgemäss in diesem mittels der Kufen ein.

Gemäss einer weiteren bevorzugten Ausführungsform weisen die Beine des Trägers zumindest je einen Vorsprung als Anschlagsnase auf, welcher federnd gelagert ist. So ist vorgesehen, dass im Bein eine Lasche ausgebildet ist, von welcher der Vorsprung umgeben ist. Der Vorsprung ist darin federnd gelagert. Alternativ dazu kann auch ein Vorsprung mit dem Bein einstückig ausgebildet oder auf dem Bein angebracht sein und das Bein selber federnd gestaltet sein. In jedem Fall ist vorgesehen, dass der Träger mit Hilfe dieses Vorsprungs in ein Trägersystem einrastbar ist.

Die Halterung ist eine Öffnung oder ein Loch mit einem Durchmesser, der grösser ist als der Schaft und kleiner als der Kopf des aufzunehmenden Hilfsmaterials, insbesondere der Schraube. Die Schraube kann so weit zwischen die Beine des Trägers einsinken, bis der Kopf der Schraube auf der Lagerauflagefläche aufliegt.

Die Ecken der Lagerfläche sind abgerundet. Es werden Spitzen und scharfe Kanten vermieden; so ist die Verletzungsgefahr insbesondere von OP-Handschuhen o.dgl. reduziert.

Die Beine bieten flächige Anschlagsabschnitte, an denen mehrere Träger hintereinander aufgereiht werden können, die sich gegenseitig stabilisieren und gruppieren. Insbesondere sind jedoch die Träger gemäss einer besonderen Ausgestaltung auch mit benachbarten Trägern über dünne Grate einstückig verbunden bzw. angespritzt und können durch Kraft vereinzelt werden,

Für die Sterilisation ist der Träger aus einem sterilisierbaren Kunststoff gefertigt. Dieser ist dabei so gewählt, dass vor allem ionisierende Strahlen-, Gas- und gegebenenfalls Dampf- Sterilisation möglich ist.

Die Abdeckung ist eine vorzugsweise gewölbte Abdeckung mit wenigstens einem Zahn, die die Lagerauflagefläche von einer kurzen Seite zur einer zweiten kurzen Seite der Lagerfläche überspannt. Der Zahn kann auch als Vorsprung bezeichnet werden. Die Wölbung der Abdeckung bietet einen Hohlraum von zumindest in der Grösse des Kopfes der aufnehmenden Schraube. Die Wölbung folgt beispielsweise einem Kreisradius, der nahezu einen Halbkreis bildet. Auch die Abdeckung ist bevorzugt aus einem durchsichtigen, sterilisierbaren Kunststoff hergestellt worden.

Die Abdeckung des Trägers hat im Bereich der höchsten Erhebung der Wölbung einen Griff, der vorzugsweise mit vier Seiten - die gegebenenfalls grifffreundlich ausgebildet sind - ausgestattet ist, und in die entgegen gesetzte Richtung als die aufzunehmende Schraube weist. Zwei Seiten, jeweils einem Zahn zugewandt, sind berandet. Aus einer Seite tritt gemäss einer besonderen Ausgestaltung ein Stöpsel hervor. An einer anderen Seite ist eine Öffnung vorgesehen. Stöpsel und Öffnung bilden ein Stecksystem.

An Stelle des Stecksystems mit Stöpsel und Öffnung ist - wie schon oben angedeutet - in einer alternativen Ausführungsform auch vorgesehen, ein Sollbruchsystem zu verwenden. Das Stecksystem ist aus einem Material gefertigt, dass an seinen Übergangsstellen, die mit dem Stöpsel und Öffnungssystem vergleichbar sind, brechen kann.

Ein solcher Träger mit oder ohne Deckel bildet den erfindungsgemässen Behälter. Dieser kann mit einem Trägersystem zusammenwirken. Vorstellbar sind verschiedene Trägersysteme. Ein solches Trägersystem hat eine ausgestreckte tablettartige Form, die in regelmässigen Abständen mit Gleitschienen versehen ist, deren Gleitflächen auf den Unterseiten vorhanden sind, und die alle parallel zueinander, insbesondere gleich beabstandet verlaufen.

Bei einem solchen Trägersystem sind ferner gemäss einer weiteren Ausgestaltung Anschlagsnasen vorgesehen, die zwischen den Gleitschienen an den Rändern des Trägersystems vorhanden sind. Solche Trägersysteme zusammen mit erfindungsgemässen Trägern sind deswegen so gestaltet, um zwischen den Gleitschienen durch vertikale Abwärtsbewegung Träger einzurasten. Die eingerasteten Träger sind in dem Trägersystem in horizontaler Richtung an den Gleitschienen verschiebbar. Sind die Träger mit Schrauben bestückt so werden sie durch die Anschlagsnase auf dem Trägersystem zurückgehalten. Wurde die Schraube jedoch dem Träger entnommen, so kann der Chirurg oder seine Assistenz den Träger leicht von dem Trägersystem nach seitlich dem Verlauf der Schiene folgend entfernen. Der leere Träger kann über die Anschlagsnase hinweg geschoben werden, während die noch bestückten Träger automatisch zurückgehalten werden.

Alternative Rückhaltesysteme sind dadurch gebildet, dass Noppen oder Verengungen im Bereich der Gleitschiene ein seitliches Entnehmen eines Trägers nur nach Kraftaufbringung gegen einen Rast- oder Reibungswiderstand ermöglichen. Bei diesen Systemen können Träger mit oder ohne Hilfsmaterial entnommen werden.

Um ein fertig bestücktes System zu verwenden, nimmt der Chirurg die separierbar verbundenen Abdeckungen als Ganzes ab. Sämtliche durch diese Abdeckung gesicherten Hilfsmaterialien sind damit zugänglich. Alternativ ist vorgesehen, dass er den Griff einer Abdeckung nimmt und so viele Abdeckungen von der Verrasterung durch die Zähne in den Rückbildungen frei dreht, wie er in dem Moment Hilfsmaterialien benötigt. Die übrigen Hilfsmaterialen bleiben dabei mittels Abdeckung verschlossen bzw. gesichert.

Ein anderes, einfacheres Trägersystem kann deutlich weniger Träger aufnehmen. Dieses Trägersystem umfasst einen U-förmigen Sockel, mit einem Hohlraum zwischen den nach innen zurückgesetzten Extremitäten, den Enden der U-Form. Ihre oberste Flächen dienen als Schlitten für die Unterseite der Lagerauflagefläche eines Trägers. Auch ein solches System kann sehr gut sterilisiert werden. Es ist kompakter und daher leichter zu spezifizieren und zusammenzustellen als das zuerst beschriebene Trägersystem. Zur Entnahme kann der Chirurg die Abdeckungen sowohl als Ganzes entnehmen als auch die Abdeckungen auf dem Schlitten verschieben und so Zugang zu nur einzelnen Hilfsmaterialien finden.

Anstelle der angegebenen geschlossenen Abdeckungen sind auch klappenähnliche Abdeckungen durch die Erfindung umfasst, die weniger eine vollständige Abdeckung, dafür jedoch eine seitlich wegschwenkbare Verlustsicherung darstellen. Diese Klappen können am Träger angelenkt oder mit diesem einstückig ausgebildet (angespritzt) sein. Sie können im Rahmen der Erfindung für einzelne Träger mit einzelnen Aufnahmen oder für Träger mit mehreren Aufnahmen vorgesehen sein. Ein solches system könnte z.B. die abnehmbaren Deckel der angegebenen Systeme von KLS Martin ersetzen. Der Vorteil bei solchen wegklappbaren Klappen liegt darin, dass sie stets am Träger verbleiben und die Manipulation somit u.U. einfacher ist, als mit Deckeln, die vollständig abnehmbar sind. Eine weitere Alternative wären Schiebedeckel.

Die Bezugszeichenliste und die Zeichnung sind zusammen mit den in den Ansprüchen beschriebenen, beziehungsweise geschützten Gegenständen integrierender Bestandteil der Offenbarung dieser Anmeldung.

### Figurenbeschreibung

Die Erfindung kann noch besser verstanden werden, indem auf die beiliegenden beispielhaften Figuren verwiesen wird. Sie werden zusammenhängend und übergreifend beschrieben. Gleiche Bezugszeichen bedeuten gleiche Bauteile, Bezugszeichen mit Apostrophen geben funktionengleiche Bauteile an.

Es zeigen dabei:
Fig. 1 eine erste Ausführungsform eines erfindungsgemässen Trägers;
Fig. 2 eine erste Ausführungsform einer Abdeckung in stehender Ansicht (A) und in liegender Ansicht (B);
Fig. 3 das Zusammenwirken des Trägers nach Fig. 1 mit einer Abdeckung nach Fig. 2 A;
Fig. 4 das Zusammenwirken von mehreren Trägern der Art nach Fig. 3;
Fig. 5 eine weitere erfindungsgemässe Abdeckung für einen Träger nach Fig. 1; jedoch mit anderen Proportionen,
Fig. 6 einen Träger nach Fig. 1 mit einer Abdeckung nach Fig. 5, die eine chirurgische Schraube aufgenommen hat;
Fig. 7 ein Trägersystem mit einzelnen Trägern nach Fig. 1 in Draufsicht;
Fig. 8 eine erste Ausführungsform eines Trägersystems nach Fig. 7 aus einer Frontsicht;
Fig. 9 eine zweite Ausführungsform eines Trägersystems nach Fig. 7 aus einer Frontsicht im Schnitt;
Fig. 10 ein Trägersystem nach Fig. 7 aus einer perspektivischen Ansicht; und
Fig. 11 ein Trägersystem nach einer dritten Ausführungsform, die kompakter ist als das Trägersystem nach Fig. 7.

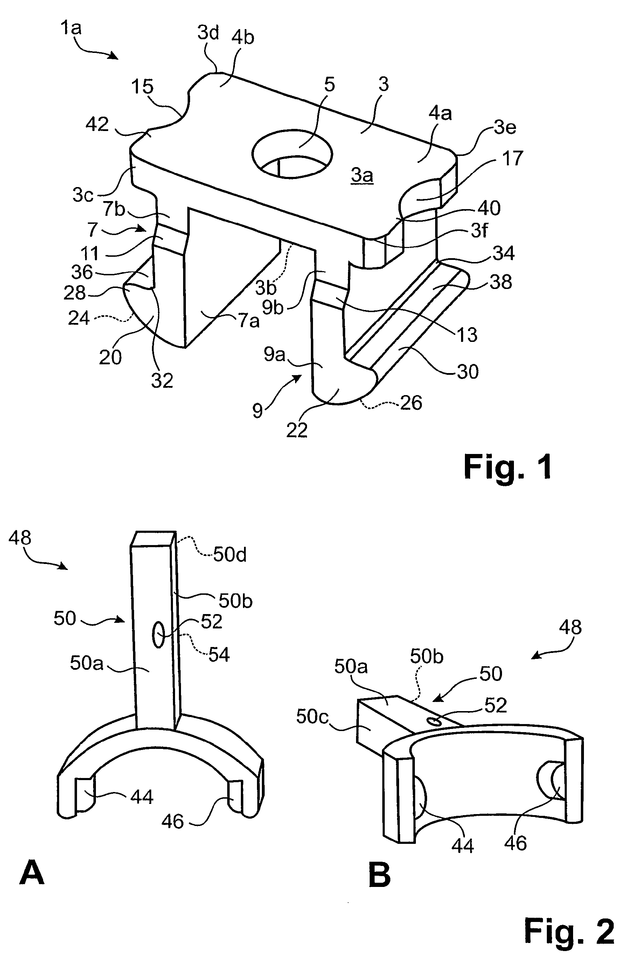

Fig. 1 bildet einen Träger 1 für eine chirurgische Schraube ab, der geeignet ist, eine chirurgische Schraube in Vereinzelung im sterilen Zustand aufzunehmen, aufzubewahren und darzubieten. Der Träger 1 umfasst eine Lagerfläche 3, an deren beiden äusseren, voneinander beabstandeten Endbereichen 4a, 4b sich jeweils ein Bein 7, 9 befindet. Jedes Bein erstreckt sich jeweils an der der Lagerauflagefläche 3a gegenüberliegenden Seite 3b, die auch als Unterseite bezeichnet wird, anfangend in nahezu rechtem Winkel zu der Richtung der Lagerfläche 3. Das erste Bein 7 hat einen oberen Bereich 7b und einen unteren Bereich 7a, das zweite Bein 9 hat einen unteren Bereich 9a und einen oberen Bereich 9b. In dem unteren Bereich 7a des ersten Beins 7 und in dem unteren Bereich 9a des zweiten Beins 9 münden das jeweilige Bein in eine Kufen 20, 22. Die erste Kufe 20 ist die Kufe für das erste Bein 7, die zweite Kufe 22 ist die Kufe für das zweite Bein 9.

Die Beine 7, 9 sind der Form nach ähnlich zueinander gestaltet, und haben in ihren oberen Bereichen 7b, 9b ungefähr die gleiche Dicke wie die Dicke der Lagerfläche 3. Nur im unteren Bereich 7a, 9a gehen die Beine 7, 9 in ihrer Dicke auseinander, um die Kufen 20, 22 zu bilden. Die Kufen 20, 22 haben Unterseiten 24, 26, die insgesamt gerundet sind und ungefähr einen Viertelkreis bilden, der in den gerundeten Übergangsbereichen 28, 30 in die Oberflächen 36, 38 übergeht. Die Kufen 20, 22 laufen entlang der Beine 7, 9 und liegen parallel zu den kurzen Seiten 40, 42 der Lagerfläche 3. Die Kufen 7, 9 haben weitere Rundungen 32, 34, die entgegengesetzt konvex zu den Übergangsbereichen 28, 30 geneigt sind.

Die meisten Ecken und Kanten des Trägers sind abgerundet, so zum Beispiel die Ecken 3c, 3d, 3e, 3f, die die Lagerauflagefläche 3a begrenzen. Hierdurch ist der Träger 1 für die Finger bzw. OP-Handschuhe eines Chirurgen und die Wunde eines Patienten verletzungsungefährlicher. Durch die Schrägen 11, 13 bilden die Beine 7, 9 in den unteren Bereichen, d.h. dort, wo die Kufen 20, 22 ausgebildet sind, hervortretende Abschnitte. Unterhalb und oberhalb der Schrägen sind die Beine 7, 9 flächig.

Die Lagerfläche 3 ist mit einem Loch 5 versehen, das in der Mitte der Lagerfläche 3 als Aussparung angeordnet sein kann. Das Loch 5 ist bevorzugt mittig angeordnet, weist also zu allen gleichartigen Rändern der Lagerfläche 3 den gleichen Abstand auf. In einer nicht dargestellten Variante ist natürlich auch vorstellbar, dass das Loch exzentrisch angeordnet ist. Das Loch 5 ist dazu bestimmt, ein Hilfsmaterial aufzunehmen. Unter Hilfsmaterial sind Schrauben, Stifte, Nieten o.dgl. zu verstehen. Die Erfindung ist darauf jedoch nicht eingeschränkt. So können unter diesen Begriff auch Bohrer, Fräser oder andere Werkzeuge fallen. Das Loch umschliesst beispielsweise bei einer chirurgischen Schraube das Schraubengewinde dieser teilweise und umrundet es. Gleichzeitig ist es so klein, dass der Schraubenkopf der chirurgischen Schraube nicht durchrutschen kann. Jeder Träger ist bevorzugt mit nur einem Loch 5 ausgestattet.

Die Träger können verschieden färbig ausgebildet sein, um so einem Chirurgen beispielsweise zu signalisieren, welche Hilfsmaterialien durch sie aufbewahrt werden. Dementsprechend können auch die Löcher unterschiedliche Grösse aufweisen.

Es können jedoch auch mehrere Löcher vorgesehen sein, insbesondere zwei, Löcher 5. In dem dargestellten bevorzugten Ausführungsbeispiel ist nur ein Loch 5 vorgesehen.

Die Seiten 40, 42 der Lagerfläche 3 haben Rückbildungen 15, 17, die ungefähr auf halber Strecke der kurzen Seiten 40, 42 platziert sind. Die Rückbildungen 15, 17 haben solche Abmessungen, dass Zähne 44, 46, die einstückig aus einer Abdeckung 48 nach Fig. 2A und Fig. 2B hervortreten, in die Rückbildungen eingreifen können, um die Abdeckung 48 an der der Lagerauflagefläche 3a gegenüberliegenden Seite 3b zu verankern.

Wie aus Fig. 2 ersichtlich, hat die Abdeckung 48 eine gewölbte Form, die den Kopf einer chirurgischen Schraube umspannen kann und gegen senkrechte Berührung schützt. In einem Bereich oberhalb eines fiktiven Mittelpunktes der Abdeckung 48, der ausserhalb des überspannten Kreisabschnittes der Wölbung liegt, befindet sich ein aus der Abdeckung heraustretender Griff 50, mit vier Seiten 50a, 50b, 50c, 50d, die paarweise gleiche Abmessungen haben können.

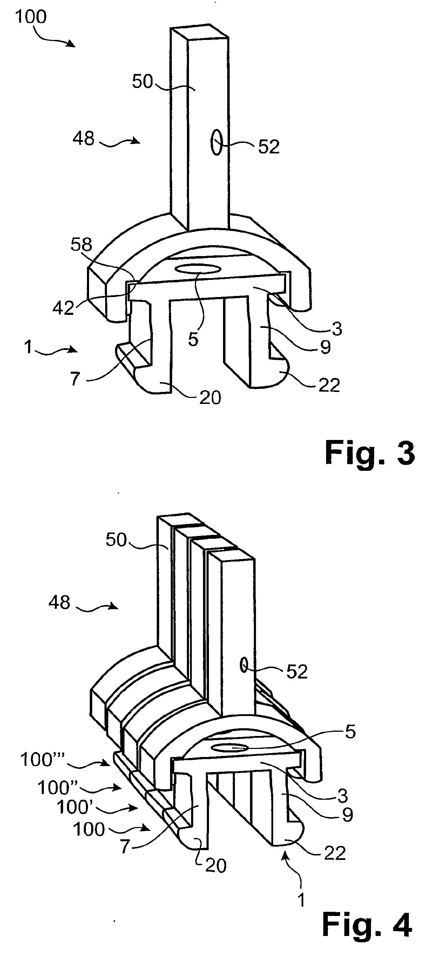

In der Fig. 3 wird das Zusammenwirken des Trägers 1 mit der Abdeckung 48 abgebildet. Träger 1 und Abdeckung 48 bilden einen kompletten Behälter 100. Die Zähne 44, 46 stehen dabei im Eingriff. Der Stöpsel 52, der aus der Seite 50a des Griffes 50 herausragt, übernimmt die Aufgabe, eine Befestigung für eine zweite Abdeckung zu bilden, so dass eine Seite eines Griffs der zweiten Abdeckung in weiten Teilen parallel zu der Seite 50a des ersten Griffs 50 liegt und gemeinsam einen grösseren Griff, der aus den Griffen 50, 50a besteht, bildet. Deutlich wird der Zustand nach dem Koppeln mehrerer Behälter 100 aus der Darstellung der Fig. 4. Dem Stöpsel 52 ist eine entsprechende Öffnung 54 auf der gegenüberliegenden Seite 50d jedes Griffes 50 zugeordnet. Die Verbindung von mehreren Behältern erfolgt demzufolge mit Hilfe der Stöpsel 52 und den korrespondierenden Öffnungen 54. Die Träger 1 werden folglich mit anderen Trägern über die Abdeckungen verbunden. Dies bringt den Vorteil mit sich, dass befüllte Behälter über die Abdeckungen zu mehreren verbunden werden können, was ihre Handhabung erleichtert. Ist die Abdeckung 48 für die Entnahme des Hilfsmaterials jedoch abgenommen worden, so kann der leere Träger 1 nun einzeln von den anderen Trägern separiert werden.

Wie oben ausgeführt sieht ein weiteres bevorzugtes Ausführungsbeispiel der Erfindung eine Sollbruchstelle zwischen den Abdeckungen bzw. den Trägern vor. Die Einheit an Abdeckungen bzw. Trägern wird dabei in einem Stück hergestellt, z. B. durch Spritzguss. Zwischen den einzelnen Elementen der Einheit wird dadurch eine schwache Verbindung geschaffen, die z. B. nur an einem Punkt besteht. So kann der Stöpsel 52 in Fig. 3 und 4 auch als diese schwache Verbindung angesehen, sprich als Sollbruchstelle. Im verbundenen Zustand sind die Abdeckungen darüber miteinander verbunden. Der Chirurg trennt die Abdeckungen jedoch dadurch, dass er die Sollbruchstelle durchtrennt.

Die Abdeckung 48 ist an ihren Innenseiten mit Auflageflächen 58 versehen, die eine Auflage der Abdeckung 48 mit den dazugehörigen Endbereichen 4a, 4b des Trägers 1 bilden können. In ihrer Mitte ragen die Zähne 44, 46 hervor. Der Behälter 100 ist gebildet, wenn die Abdeckung 48 über die Zähne 44, 46 fest mit dem Träger 1 im Bereich der Rückbildungen 15, 17 an den kurzen Seiten 40, 42 im Eingriff steht und die Abdeckung 48 auf ihren Auflageflächen 58 ruht. Wichtig für das Sterilisieren ist, dass die chirurgische Schraube trotz Abdeckung weiterhin gut zugänglich ist, so dass sterilisierender Dampf ein- und austreten kann.

Mit diesem Stecksystem Stöpsel 52 und Öffnung 54 wird ein zusammensteckbares System, so wie in Fig. 4 dargestellt, für zahlreiche chirurgische Schrauben geschaffen, die jeweils gegen senkrechtes Berühren geschützt sind. Die Schrauben liegen jedoch pro Träger 1 in jedem Behälter 100, 100', 100", 100"' vereinzelt und unverlierbar vor.

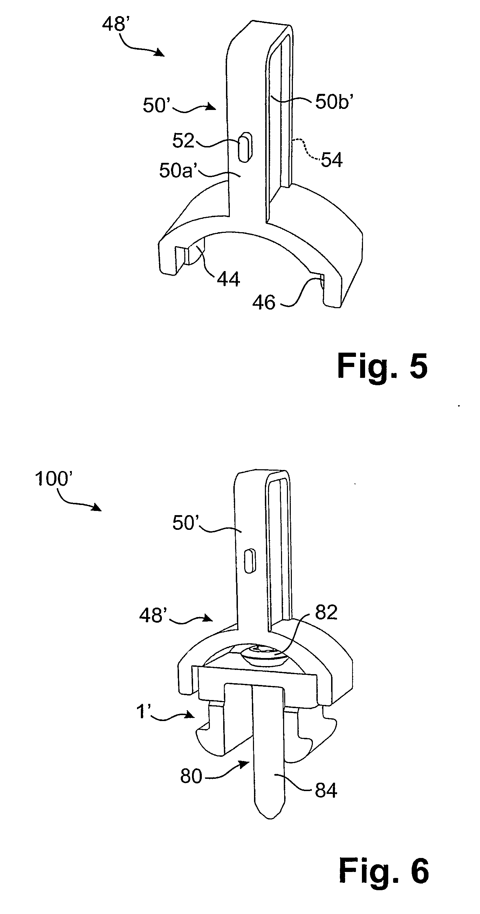

Fig. 5 stellt einen weitere, erfindungsgemässe Abdeckung 48' dar, die in weiten Teilen ähnlich zu einer Abdeckung nach Fig. 2A oder Fig. 2B ist. Die Abdeckung 48' hat zwei Zähne 44, 46, einen ersten Zahn 44 und einen zweiten Zahn 46, die so gestaltet sind, dass sie mit entsprechenden Rückbildungen 15, 17, wie zum Beispiel in Fig. 1 dargestellt, zusammenpassen und einrasten können. Der Griff 50' der Abdeckung 48' hat eine andere Form als der Griff 50 nach Fig. 2A. In der Fig. 5 sind von dem Griff 50' die beiden Seiten 50a' und 50b' zu sehen. Die Seite 50b' ist berandet. Der Rand hat die Wirkung, dass die Finger eines Nutzers besser den Griff 50' angreifen können. Zur weiteren Verbesserung der Greifbarkeit des Griffs 50' kann die Oberfläche gerippt oder mit Noppen o.dgl. versehen sein. Die Seite 50a' und die dieser Seite gegenüberliegende Seite können ausserdem beschriftet oder - beispielsweise maschinenlesbar - codiert werden. Auch bietet es sich an, die Trägerfläche 3 zu beschriften, z. B. mit Längenangaben der Schrauben.

In Fig. 6 ist eine Abdeckung 48' mit Griff 50' mit einem Träger 1' zu sehen, der ein chirurgisches Hilfsmittel, wie eine stilistisch dargestellte Schraube 80, trägt. Die Schraube 80 umfasst einen Schraubenkopf 82 und einen Schraubenschaft 84, der im Loch lose hängt.

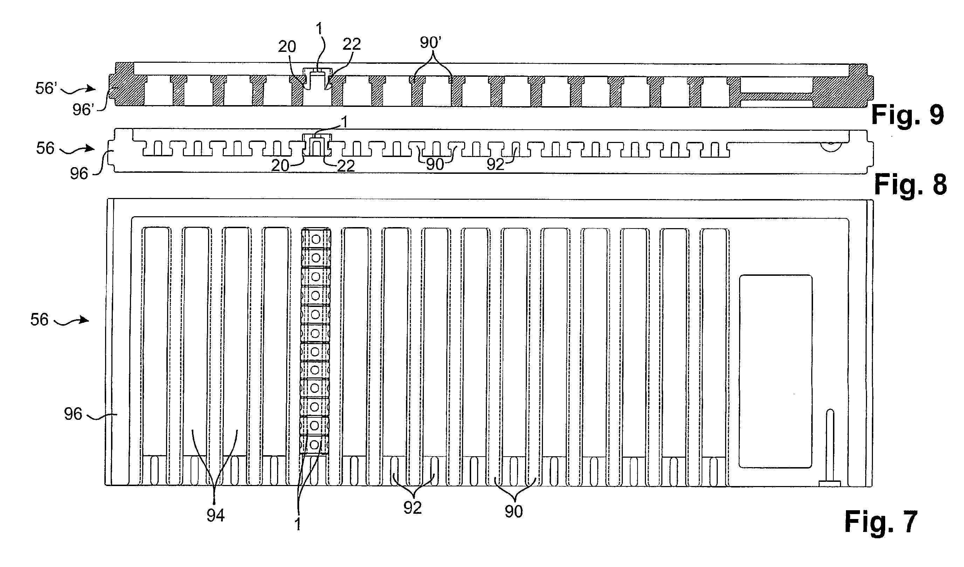

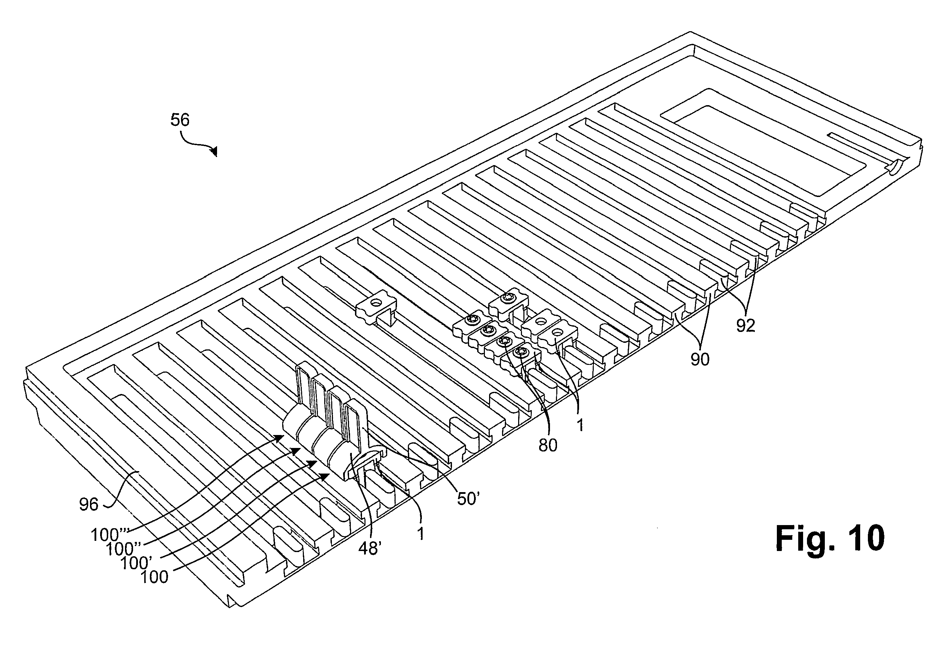

Fig. 7 stellt ein Trägersystem 56 aus einer Draufsicht dar. In Fig. 8 ist dieses im Querschnitt dargestellt und in Fig. 10 in perspektivischer Darstellung. Fig. 9 stellt einen Schnitt durch ein (weiteres) Trägersystem 56' dar. Das Trägersystem 56, 56' ist mit Gleitschienen 90, 90' ausgestattet, die zueinander über einen Freiraum 94 beabstandet sind. Der Freiraum hat die Abmessungen, dass ein Träger 1 zwischen den Gleitschienen 90, 90' eingreifen kann. An einem Ende jedes Freiraums 94 sind jeweils eine Anschlagsnase 92 vorgesehen. Die Träger 1 halten sich im normalen bevorratenden Zustand in dem Bereich des Freiraums 94 auf, der hinter den Anschlagsnasen 92 beginnt. Das Trägersystem 56, 56' hat einen an drei Seiten umlaufenden Rand 96, 96'.

Die beiden Ausgestaltungen der Fig. 7, 8 und 10 einerseits und der Fig. 9 andererseits unterscheiden sich unter anderem dadurch, dass die Gleitschienen 90, 90' unterschiedliche Höhen haben. In den Fällen, in denen Träger mit langen Schrauben 80 vorbereitet werden sollen, ist ein Trägersystem 56 mit höheren Gleitschienen 90' zu wählen, während in den Fällen, in denen nur kurze Schrauben 80 zum Einsatz kommen, ein Trägersystem 56 mit kürzeren Gleitschienen 90 einzusetzen ist. Auch ist es vorstellbar, ein modifiziertes Trägersystem 56 zu haben, dass eine gewisse Anzahl hoher Gleitschienen 90' und eine gewisse Anzahl kurzer Gleitschienen 90 hat - jeweils für die Aufnahme von unterschiedlichen Trägern oder Trägern mit unterschiedlichen Hilfsmitteln.

Die Trägersysteme 56, 56' sind so mit einem Schutzrahmen 96, 96' versehen, dass der Rahmen höher baut, als die Lagerauflagefläche 3a der Träger 1 nach oben ragt. Die Kufen 20, 22 greifen seitlich unter die Gleitschienen 90, 90' und gewähren so einen verschiebbaren, einrastbaren Mechanismus, so dass die Träger nach dem Einrasten nicht in vertikaler Richtung weg von dem Trägersystem entnehmbar sind, aber in einer horizontalen, parallel zu den Gleitschienen 90, 90' gegebenen Richtung verschiebbar sind.

In Fig. 10 ist das Trägersystem 56 aus Fig. 7 in einer perspektivischen Sicht zu sehen. Das Trägersystem 56 trägt verschiedene Träger 1, die teilweise leer sind, teilweise Abdeckungen 48' haben und teilweise mit Schrauben 80 versehen sind. Die Anschlagsnasen 92 halten die Träger 1 zurück, die mit Schrauben 80 ausgestattet sind, weil sie den freilaufenden horizontalen Weg aus dem Trägersystem 56 durch einen Anschlag der Schrauben an den Anschlagsnasen 92 verhindern. Die Abdeckungen 48 mit ihren Griffen 50 lassen die vereinzelten Schrauben 80 leicht hin- und herrutschen, indem z.B. ein Chirurg nur nach einer Abdeckung greifen muss und durch das System aus Stöpsel und Öffnung dann eine gesamte vorbereitete Gruppe von mehreren Behältern 100, 100', 100", 100"' entlang den Gleitschienen 90, 90' bewegen kann.

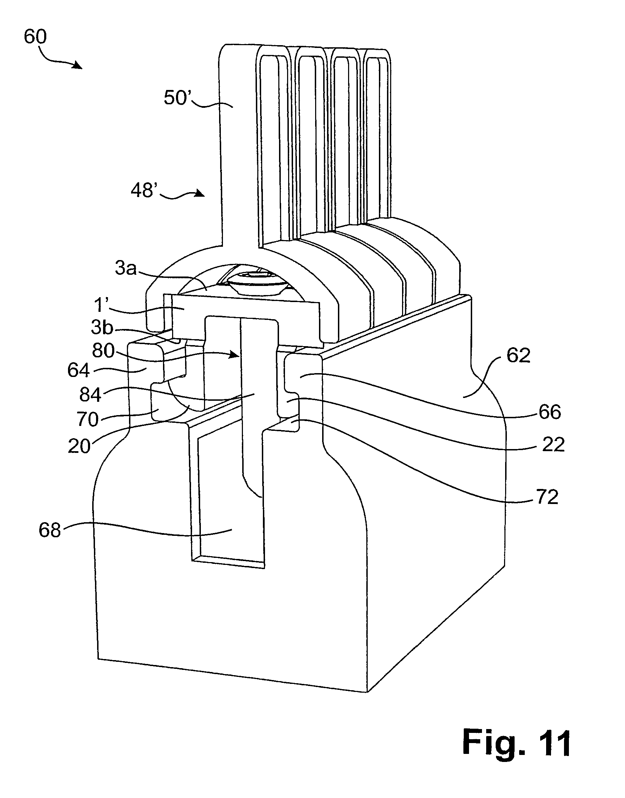

In Fig. 11 ist ein möglicher Haltekörper 60 offenbart, der aus einem Schlitten 62 und wenigstens je einem Träger 1' und einer Abdeckung 48' besteht. Der Schlitten 62 hat die Form einer Klammer bzw. die Form eines gängigen Clips, der ein U-Stück bildet, und an seinen Extremitäten 64, 66 die Seite 3b des Trägers 1', die der Lagerauflagefläche 3a gegenüberliegt, berührt und hält. Der Hohlraum 68 des U-Stücks ist in seiner Höhe und Breite auf den Träger 1 abgestimmt, so dass eine Schraube 80 in dem Hohlraum 68 aufgenommen werden kann. Auf den Innenseiten der Extremitäten 64, 66, sind Einkerbungen 70, 72 vorhanden, die für die Stabilität des Schlitten 62 sorgen und gleichzeitig den Platz bieten, so dass die Kufen 20, 22 in den Einkerbungen gleiten können.

Eine weitere alternative Ausführungsform eines Trägers sieht vor, dass dieser nur ein Bein bzw. unterschiedlich gestaltete Beine aufweist. So kann das eine Bein als ein solches ausgebildet sein und sich beispielsweise in dem Trägersystem abstützen. Das andere "Bein" ist als Eingriffselement ausgestaltet, das unter die Gleitschienen eingreift und in das Trägersystem einrastet. Ein solcher Träger lässt sich leichter aus den Gleitschienen nach oben - also gegen die Einsetzrichtung - wieder entfernen bzw. ausklipsen.

Die Träger sind aus sterilisierbarem Kunststoff gefertigt, und können farblich codiert sein. An Hand des Farbcodes kann der Chirurg erkennen, welche Schraube er vor sich hat. Besonders vorteilhaft ist es, wenn der Träger einen leicht zu erkennenden Farbcode, wie zum Beispiel eine komplette Einfärbung trägt, während die Abdeckung aus einem durchsichtigen Kunststoff hergestellt ist. Der durchsichtige Kunststoff lässt zu, dass der Nutzer, z. B. der Chirurg, leicht einen Blick auf die abgedeckte Schraube werfen kann, und sich so noch einmal vergewissern kann, ob sich unterhalb der Abdeckung tatsächlich auch das Hilfsmaterial für seine Operation befindet, das er dort erwartet vorzufinden. Weil die Träger und das Trägersystem im Kunststoffspritzguss herstellbar sind, kann das System sehr kostengünstig hergestellt werden.

### Bezugszeichenliste

- 1 -: Träger
- 3 -: Lagerfläche
- 3a -: Lagerauflagefläche
- 3b -: gegenüberliegende Seite
- 3c -: erste Ecke
- 3d -: zweite Ecke
- 3e -: dritte Ecke
- 3f -: vierte Ecke
- 4a -: erster Endbereich
- 4b -: zweiter Endbereich
- 5 -: Loch
- 7 -: erstes Bein
- 7a -: unterer Bereich
- 7b -: oberer Bereich
- 9 -: zweites Bein
- 9a -: unterer Bereich
- 9b -: oberer Bereich
- 11 -: Schräge
- 13 -: Schräge
- 15 -: Rückbildung
- 17 -: Rückbildung
- 20 -: erste Kufe
- 22 -: zweite Kufe
- 24 -: erste Unterseite
- 26 -: zweite Unterseite
- 28 -: erster Übergangsbereich
- 30 -: zweiter Übergangsbereich
- 32 -: erste Rundung
- 34 -: zweite Rundung
- 36 -: erste Oberfläche
- 38 -: zweite Oberfläche
- 40 -: erste kurze Seite
- 42 -: zweite kurze Seite
- 44 -: erster Zahn
- 46 -: zweiter Zahn
- 48 -: Abdeckung
- 50 -: G riff
- 50a -: erste Seite des Griffs
- 50b -: zweite Seite des Griffs
- 50c -: dritte Seite des Griffs
- 50d-: vierte Seite des Griffs
- 52 -: Stöpsel
- 54 -: Öffnung
- 56 -: Trägersystem
- 58 -: Auflagefläche
- 60 -: Haltekörper
- 62 -: Schlitten
- 64 -: erste Extremität
- 66 -: zweite Extremität
- 68 -: Hohlraum
- 70 -: erste Einkerbung
- 72 -: zweite Einkerbung
- 80 -: Schraube
- 82 -: Schraubenkopf
- 84 -: Schraubenschaft
- 90 -: Gleitschiene
- 92 -: Anschlagsnase
- 94 -: Freiraum
- 96 -: Schutzrahmen
- 100-: Behälter

## Patentansprüche

1. Behälter (100), der einen Träger (1) für die Lagerung und Darbietung von chirurgischem Hilfsmaterial (80), insbesondere Schrauben wie Knochenschrauben, in Aufnahme- bzw. Montagelage, und zumindest eine abnehmbare Abdeckung (48) zur Verlustsicherung des Hilfsmaterials aufweist, wobei das chirurgische Hilfsmaterial (80) vereinzelt darbietbar ist, und wobei zumindest zwei Behälter (100) miteinander separierbar verbunden sind, sei es über die Abdeckung (48) und/oder über den Träger (1),
**dadurch gekennzeichnet, dass** das chirurgische Hilfsmaterial (80) **dadurch** vereinzelt darbietbar ist, dass der Träger (1) in Form eines Tisches eine Lagerauflagefläche (3, 3a) mit einer Halterung in Form eines Lochs (5), aufweist, an welcher Lagerauflagefläche (3, 3a) das chirurgische Hilfsmaterial (80) zu halten ist, derart, dass es von der Lagerauflagefläche nach unten hängt und jede einzelne Aufnahmeposition in vereinzelnder Form am Träger (1) nur zur Aufnahme eines einzigen, sich von der Lagerfläche (3, 3a) in die Halterung (5) hängenden Hilfsmaterials (80) ausgebildet ist und/oder ihr jeweils nur eine abnehmbare Abdeckung (48) zugeordnet ist.

2. Behälter (100) nach Anspruch 1, **dadurch gekennzeichnet, dass** das chirurgische Hilfsmaterial (80) **dadurch** vereinzelt darbietbar ist, dass für jede einzelne Aufnahmeposition eine abnehmbare Abdeckung (48) vorgesehen ist.

3. Behälter (100) nach Anspruch 1, **dadurch gekennzeichnet, dass** das chirurgische Hilfsmaterial (80) **dadurch** vereinzelt darbietbar ist, dass der Träger (1) nur eine Aufnahmeposition zur Aufnahme eines einzigen Hilfsmaterials (80) aufweist.

4. Behälter (100) nach einem der vorhergehenden Ansprüche, **dadurch gekennzeichnet, dass** die zumindest zwei Behälter (100) über deren Abdeckungen (48) separierbar verbunden sind.

5. Behälter (100) nach einem der vorhergehenden Ansprüche, **dadurch gekennzeichnet, dass** die zumindest zwei Behälter (100) über deren Träger (1) separierbar verbunden sind.

6. Behälter (100) nach einem der vorhergehenden Ansprüche, **dadurch gekennzeichnet, dass** zwischen benachbarten und verbundenen Trägern oder benachbarten und verbundenen Abdeckungen eine Sollbruchstelle vorgesehen ist, so dass Träger (1) und/oder Abdeckung (48) separierbar sind.

7. Behälter (100) nach einem der Ansprüche 1 - 5, **dadurch gekennzeichnet, dass** zwischen benachbarten und verbundenen Trägern oder benachbarten und verbundenen Abdeckungen ein lösbares Verbindungsmittel vorgesehen ist, insbesondere eine Öffnung (54) und ein Stöpsel (52), so dass Träger (1) und/oder Abdeckung (48) separierbar sind.

8. Behälter (100) nach einem der vorhergehenden Ansprüche, **dadurch gekennzeichnet, dass** das chirurgische-Hilfsmaterial (80) eine einzige Schraube oder ein Schraubenpaar ist.

9. Behälter nach einem der vorhergehenden Ansprüche, **dadurch gekennzeichnet, dass** der Träger (1) mit wenigstens einem oder vorzugsweise zwei Beinen (7, 9) ausgestattet ist, die im Wesentlichen im rechten Winkel aus der der Lagerauflagefläche (3a) gegenüberliegenden Seite (3b) hervorragen.

10. Behälter nach Anspruch 9, **dadurch gekennzeichnet, dass** der Träger (1) eine Halterung in Form eines Lochs (5) aufweist, in der nur ein einziges vereinzeltes Hilfsmaterial (80) mit einem Kopf und einem Schaft, insbesondere eine vereinzelte Schraube oder ein Schraubenpaar, aufgenommen werden kann, dessen Kopf sich auf der Lagerauflagefläche befindet und dessen Schaft zwischen den Beinen (7, 9) parallel zu den Beinen steckt.

11. Behälter (100) nach Anspruch 10, **dadurch gekennzeichnet, dass** die Halterung des Trägers (1) mindestens ein Loch (5) mit einem Durchmesser ist, der grösser ist als der Schaft und kleiner als der Kopf des aufzunehmenden Hilfsmittels bzw. der aufzunehmenden Schraube (80).

12. Behälter nach einem der vorhergehenden Ansprüche, **dadurch gekennzeichnet, dass** die Lagerauflagefläche (3) mit wenigstens einer Rückbildung (15, 17) versehen ist, die so bemessen ist, dass ein passender Rast-Zahn (44, 46) der Abdeckung (48) über die Rückbildung (15, 17) mit der der Lagerauflagefläche (3a) gegenüberliegende Seite (3b) verriegeln kann.

13. Behälter (100) nach einem der Ansprüche 9 bis 12, **dadurch gekennzeichnet, dass** die Beine (7, 9) des Trägers (1) in Kufen (20, 22) münden, die insgesamt eine gerundete Form aufweisen, und lateral nach Aussen wegdeuten.

14. Behälter (100) nach einem der Ansprüche 9 bis 12, **dadurch gekennzeichnet, dass** die Beine (7, 9) des Trägers (1) in Kufen (20, 22) münden, die insgesamt lateral nach Innen hineindeuten.

15. Behälter (100) nach einem der Ansprüche 9 bis 12, **dadurch gekennzeichnet, dass** die Beine (7, 9) des Trägers (1) zumindest je einen Vorsprung als Anschlagsnase aufweisen, der federnd gelagert ist.

16. Behälter (100) nach einem der vorhergehenden Ansprüche, **dadurch gekennzeichnet, dass** Kanten und/oder Ecken (3c, 3d, 3e, 3f) der Lagerfläche (3, 3a) und/oder der Abdeckung (48) abgerundet sind.

17. Behälter (100) nach einem der Ansprüche 9 bis 16, **dadurch gekennzeichnet, dass** die Beine (7, 9) flächige Anschlagsabschnitte oder Führungsflächen bieten.

18. Behälter (100) nach einem der vorhergehenden Ansprüche, **dadurch gekennzeichnet, dass** der Träger (1) aus einem sterilisierbaren Kunststoff gefertigt ist.

19. Behälter (100) nach einem der vorhergehenden Ansprüche, **dadurch gekennzeichnet, dass** die Abdeckung (48) eine gewölbte Abdeckung (48) ist, die mit wenigstens einem Rast-Zahn (44, 46) versehen ist, und die vorzugsweise die Lagerauflagefläche (3a) von einer kurzen Seite (40) zur einer zweiten kurzen Seite (42) der Lagerfläche (3) überspannt.

20. Behälter (100) nach einem der vorhergehenden Ansprüche, **dadurch gekennzeichnet, dass** die Wölbung der Abdeckung (48) einen Hohlraum in zumindest der Grösse des Kopfes der aufzunehmenden Schraube (80) bietet, wobei die Wölbung insbesondere einem Kreisradius folgt, der nahezu einen Halbkreis bildet.

21. Behälter (100) nach Anspruch 20, **dadurch gekennzeichnet, dass** die Abdeckung den Hohlraum **dadurch** bildet, dass die Abdeckung eine Verlängerung des Trägers (1) darstellt.

22. Behälter (100) nach einem der vorhergehenden Ansprüche, **dadurch gekennzeichnet, dass** die Abdeckung (48) als schwenkbare Klappe mit einer bügelförmigen Ausnahme in zumindest der Höhe des Kopfes der aufzunehmenden Schraube (80) ausgebildet ist.

23. Behälter nach Anspruch 22, **dadurch gekennzeichnet, dass** die Abdeckung (48) schwenkbar am Träger (1) und vorzugsweise mit dem Hilfsmittel oder den Hilfsmitteln verrastbar gelagert ist.

24. Behälter nach einem der vorhergehenden Ansprüche, **dadurch gekennzeichnet, dass** die Abdeckung (48) mit dem Träger (1) einstückig und federelastisch verbunden ist.

25. Behälter (100) nach einem der vorhergehenden Ansprüche, **dadurch gekennzeichnet, dass** die Abdeckung (48) aus einem durchsichtigen, sterilisierbaren Kunststoff hergestellt worden ist.

26. Behälter (100) nach einem der Ansprüche, **dadurch gekennzeichnet, dass** im Bereich der höchsten Erhebung der Wölbung der Abdeckung (48) ein Griff (50) mit vier Seiten (50a, 50b, 50c, 50d) vorgesehen ist, der in die entgegengesetzte Richtung als die aufzunehmende Schraube (80) weist.

27. Behälter (100) nach Anspruch 26, **dadurch gekennzeichnet, dass** zwei Seiten (50b, 50d), die insbesondere jeweils einem Zahn (44, 46) zugewandt sind, berandet oder geriffelt sind.

28. Behälter (100) nach einem der Ansprüche 26 oder 27, **dadurch gekennzeichnet, dass** eine Seite (50a) einen Stöpsel (52) aufweist, und dass eine andere Seite (50c) eine Öffnung (54) aufweist, wobei Stöpsel (52) und Öffnung (54) in einem Stecksystem zusammenwirken.

29. Behälter (100) nach einem der vorhergehenden Ansprüche, **dadurch gekennzeichnet, dass** für jede einzelne Aufnahmeposition zur Aufnahme eines einzigen Hilfsmaterials (80) in einem Behälter, in dem mehrere Hilfsmaterialien aufnehmbar sind, eine eigene abnehmbare Abdeckung (48) vorgesehen ist, wobei die einzelnen Abdeckungen miteinander separierbar verbunden sind.

## Claims

1. Container (100), which has a carrier (1) for the storage and presentation of surgical auxiliary material (80), in particular screws such as bone screws, in the holding or mounting position, and at least one removable cover (48) for preventing the loss of the auxiliary material, wherein the surgical auxiliary material (80) is able to be presented in isolated form, and wherein at least two containers (100) are separably connected with each other, whether via the cover (48) and/or via the carrier (1),
**characterized in that** the surgical auxiliary material (80) is able to be presented in isolated form **in that** the carrier (1) in the form of a table has a storage support area (3, 3a) with a mounting in the form of a hole (5), on which storage support area (3, 3a) the surgical auxiliary material (80) is to be held, such that it hangs down from the storage support area, and each individual holding position in isolated form is formed on the carrier (1) only to receive a single item of auxiliary material (80) hanging from the storage area (3, 3a) into the mounting (5), and/or only one removable cover (48) is associated with it respectively.

2. Container (100) according to Claim 1, **characterized in that** the surgical auxiliary material (80) is presentable in isolated form **in that** for each individual holding position, a removable cover (48) is provided.

3. Container (100) according to Claim 1, **characterized in that** the surgical auxiliary material (80) is presentable in isolated form **in that** the carrier (1) only has one holding position to hold a single item of auxiliary material (80).

4. Container (100) according to one of the preceding claims, **characterized in that** the at least two containers (100) are separably connected via their covers (48).

5. Container (100) according to one of the preceding claims, **characterized in that** the at least two containers (100) are separably connected via their carriers (1).

6. Container (100) according to one of the preceding claims, **characterized in that** a predetermined breaking point is provided between adjacent and connected carriers or adjacent and connected covers, so that carrier (1) and/or cover (48) are separable.

7. Container (100) according to one of Claims 1-5, **characterized in that** between adjacent and connected carriers or adjacent and connected covers, a detachable connecting means is provided, in particular an opening (54) and a stopper (52), so that carrier (1) and/or cover (48) are separable.

8. Container (100) according to one of the preceding claims, **characterized in that** the surgical auxiliary material (80) is a single screw or a pair of screws.

9. Container according to one of the preceding claims, **characterized in that** the carrier (1) is equipped with at least one or preferably two legs (7, 9), which project substantially at right angles out of the side (3b) lying opposite the storage support area (3a).

10. Container according to Claim 9, **characterized in that** the carrier (1) has a mounting in the form of a hole (5), in which only one single isolated item of auxiliary material (80) with a head and a shaft, in particular an isolated screw or a pair of screws, can be held, the head of which is situated on the storage support area and the shaft of which is placed between the legs (7, 9) parallel to the legs.

11. Container (100) according to Claim 10, **characterized in that** the mounting of the carrier (1) is at least one hole (5) with a diameter which is greater than the shaft and smaller than the head of the item of auxiliary material which is to be held or respectively screw (80) which is to be held.

12. Container according to one of the preceding claims, **characterized in that** the storage support area (3) is provided with at least one recessed area (15, 17), which is dimensioned so that a fitting detent tooth (44, 46) of the cover (48) can lock via the recessed area (15, 17) with the side (3b) lying opposite the storage support area (3a).

13. Container (100) according to one of Claims 9 to 12, **characterized in that** the legs (7, 9) of the carrier (1) open out in runners (20, 22), which as a whole have a rounded shape, and point laterally away towards the exterior.

14. Container (100) according to one of Claims 9 to 12, **characterized in that** the legs (7, 9) of the carrier (1) open out in runners (20, 22), which as a whole point laterally inwards.

15. Container (100) according to one of Claims 9 to 12, **characterized in that** the legs (7, 9) of the carrier (1) have at least one protection in each case as a stop lug, which is mounted elastically.

16. Container (100) according to one of the preceding claims, **characterized in that** edges and/or corners (3c, 3d, 3e, 3f) of the storage area (3, 3a) and/or of the cover (48) are rounded.

17. Container (100) according to one of Claims 9 to 16, **characterized in that** the legs (7, 9) offer flat stop sections or guide faces.

18. Container (100) according to one of the preceding claims, **characterized in that** the carrier (1) is produced from a sterilisable plastic.

19. Container (100) according to one of the preceding claims, **characterized in that** the cover (48) is a curved cover (48), which is provided with at least one detent tooth (44, 46), and which preferably spans the storage support area (3a) from a short side (40) to a second short side (42) of the storage area (3).

20. Container (100) according to one of the preceding claims, **characterized in that** the curvature of the cover (48) offers a cavity of at least the size of the head of the screw (80) which is to be held, wherein the curvature in particular follows a circle radius which almost forms a semicircle.

21. Container (100) according to Claim 20, **characterized in that** the cover forms the cavity **in that** the cover constitutes an extension of the carrier (1).

22. Container (100) according to one of the preceding claims, **characterized in that** the cover (48) is constructed as a pivotable flap with a U-shaped recess at at least the height of the head of the screw (80) which is to be held.

23. Container according to Claim 22, **characterized in that** the cover (48) is mounted pivotably on the carrier (1) and preferably so as to be able to be engaged with the single or multiple auxiliary means.

24. Container according to one of the preceding claims, **characterized in that** the cover (48) is connected with the carrier (1) in one piece and in a spring-elastic manner.

25. Container (100) according to one of the preceding claims, **characterized in that** the cover (48) has been produced from a transparent, sterilisable plastic.

26. Container (100) according to one of the claims, **characterized in that** in the region of the highest elevation of the curvature of the cover (48), a handle (50) with four sides (50a, 50b, 50c, 50d) is provided, which points in the opposite direction than the screw (80) which is to be held.

27. Container (100) according to Claim 26, **characterized in that** two sides (50b, 50d), which in particular in each case face a tooth (44, 46), are edged or fluted.

28. Container (100) according to one of Claims 26 or 27, **characterized in that** one side (50a) has a stopper (52), and that another side (50c) has an opening (54), wherein the stopper (52) and opening (54) cooperate in a connector system.

29. Container (100) according to one of the preceding claims, **characterized in that** for each individual holding position to hold a single item of auxiliary material (80) in a container, in which several auxiliary materials are able to be held, its own removable cover (48) is provided, wherein the individual covers are separably connected with each other.

## Revendications

1. Récipient (100) présentant un support (1) pour stocker et distribuer du matériel chirurgical auxiliaire (80) en particulier des vis comme des vis pour os, en position de réception respectivement de montage, et au moins un couvercle amovible (48) pour éviter de perdre du matériel auxiliaire, sachant que le matériel chirurgical auxiliaire (80) peut être distribué de manière individuelle, et sachant qu'au moins deux récipients (100) sont reliés l'un à l'autre en étant séparables, que ce soit par le couvercle (48) et/ou par le support (1),
**caractérisé en ce que** le matériel chirurgical auxiliaire (80) peut être distribué de manière individuelle **en ce que** le support (1) sous forme de table présente une surface d'appui de stockage (3, 3a) avec une fixation en forme de trou (5), sur laquelle surface d'appui de stockage (3, 3a) le matériel chirurgical auxiliaire (80) doit être maintenu de telle manière qu'il pende vers le bas depuis la surface d'appui de stockage (3, 3a) et chaque position de réception individuelle est formée de manière individuelle sur le support (1) uniquement pour recevoir un seul matériel auxiliaire (80) suspendu depuis la surface d'appui de stockage (3, 3a) dans la fixation (5) et/ou dispose d'un seul couvercle amovible (48).

2. Récipient (100) selon la revendication 1, **caractérisé en ce que** le matériel chirurgical auxiliaire (80) peut être distribué de manière individuelle **en ce que** pour chaque position de réception individuelle, un couvercle amovible (48) est prévu.

3. Récipient (100) selon la revendication 1, **caractérisé en ce que** le matériel chirurgical auxiliaire (80) peut être distribué de manière individuelle **en ce que** le support (1) ne présente qu'une seule position de réception pour recevoir un seul matériel auxiliaire individuel (80).

4. Récipient (100) selon l'une des revendications précédentes, **caractérisé en ce que** les au moins deux récipients (100) sont reliés de manière séparable par leurs couvercles (48).

5. Récipient (100) selon l'une des revendications précédentes, **caractérisé en ce que** les au moins deux récipients (100) sont reliés de manière séparable par leurs supports (1).

6. Récipient (100) selon l'une des revendications précédentes, **caractérise en ce qu**'entre des supports adjacents et reliés ou des couvercles adjacents et reliés, un point de rupture théorique est prévu de façon à ce que le support (1) et/ou le couvercle (48) soient séparables.

7. Récipient (100) selon l'une des revendications 1 à 5, **caractérisé en ce qu'**entre des supports adjacents et reliés ou des couvercles adjacents et reliés, un moyen de liaison séparable est prévu, en particulier une ouverture (54) et un bouchon (52) de façon à ce que le support (1) et/ou le couvercle (48) soient séparables.

8. Récipient (100) selon l'une des revendications précédentes, **caractérisé en ce que** le matériel chirurgical auxiliaire (80) est une vis unique ou une paire de vis. '

9. Récipient selon l'une des revendications précédentes, **caractérisé en ce que** le support (1) est muni d'au moins une ou de préférences deux jambes (7, 9) qui font saillie essentiellement dans l'angle droit en sortant du côté (3b) faisant face à la surface d'appui de stockage (3a).

10. Récipient selon la revendication 9, **caractérisé en ce que** le support (1) présente une fixation en forme de trou (5) dans laquelle un seul matériel auxiliaire chirurgical (80) individuel avec une tête et une tige, en particulier une vis individuelle ou une paire de vis, peut être reçue, dont la tête se trouve sur la surface d'appui de stockage et dont la tige se trouve entre les jambes (7, 9) et parallèle à celles-ci.

11. Récipient (100) selon la revendication 10, **caractérisé en ce que** la fixation du support (1) présente au moins un trou (5) avec un diamètre qui est supérieur à la tige et intérieur à la tête du matériel auxiliaire à recevoir, respectivement de la vis à recevoir (80).

12. Récipient selon l'une des revendications précédentes, **caractérisé en ce que** la surface d'appui de stockage (3) est munie au moins d'une régression (15, 17) qui est ainsi dimensionnée qu'une dent d'encliquetage adaptée (44, 46) du couvercle (48) peut se verrouiller par la régression (15, 17) avec le côté (3b) faisant face à la surface d'appui de stockage (3a).

13. Récipient (100) selon l'une des revendications 9 à 12, **caractérisé en ce que** les jambes (7, 9) du support (1) débouchent dans des patins (20, 22) qui présentent une forme arrondie et convergent latéralement vers l'extérieur.

14. Récipient (100) selon l'une des revendications 9 à 12, **caractérisé en ce que** les jambes (7, 9) du support (1) débouchent dans des patins (20, 22) qui convergent latéralement vers l'intérieur.

15. Récipient (100) selon l'une des revendications 9 à 12, **caractérisé en ce que** les jambes (7, 9) du support (1) présentent au moins une saillie respective en tant qu'ergot de butée placé de manière élastique.

16. Récipient (100) selon l'une des revendications précédentes, **caractérisé en ce que** des arêtes et/ou des angles (3c, 3d, 3e, 3f) de la surface de stockage (3, 3a) et/ou du couvercle (48) sont arrondi(e)s.

17. Récipient (100) selon l'une des revendications 9 à 16, **caractérisé en ce que** les jambes (7, 9) offrent des sections de butée ou des surfaces de guidage planes.

18. Récipient (100) selon l'une des revendications précédentes, **caractérisé en ce que** le support (1) est fabriqué dans un plastique stérilisable.

19. Récipient (100) selon l'une des revendications précédentes, **caractérisé en ce que** le couvercle (48) est un couvercle bombé (48) muni au moins d'une dent d'encliquetage (44, 46) et qui enjambe de préférence la surface d'appui de stockage (3a) depuis un côté court (40) vers un deuxième côté court (42) de la surface de stockage (3).

20. Récipient (100) selon l'une des revendications précédentes, **caractérisé en ce que** le bombement du couvercle (48) offre une cavité d'au moins la taille de la tête de la vis (80) à recevoir, sachant que le bombement forme en particulier un rayon de cercle qui forme presque un demi-cercle.

21. Récipient (100) selon la revendication 20, **caractérisé en ce que** le couvercle forme ainsi la cavité **en ce que** le couvercle représente un prolongement du support (1).

22. Récipient (100) selon l'une des revendications précédentes, **caractérisé en ce que** le couvercle (48) est formé comme un volet pivotant avec une réception en forme d'étrier d'au moins la hauteur de la tête de la vis (80) à recevoir.

23. Récipient selon la revendication 22, **caractérisé en ce que** le couvercle (48) est placé de manière pivotante sur le support (1) et est de préférence encliquetable avec le matériel auxiliaire.

24. Récipient selon l'une des revendications précédentes, **caractérisé en ce que** le couvercle (48) est relié au support (1) en étant monobloc et élastique.

25. Récipient (100) selon l'une des revendications précédentes, **caractérisé en ce que** le couvercle (48) est fabriqué dans un plastique translucide stérilisable.

26. Récipient (100) selon l'une des revendications précédentes, **caractérisé en ce qu'**au niveau du soulèvement le plus haut du bombement du couvercle (48), une poignée (50) avec quatre côtés (50a, 50b, 50c, 50d) est prévue, laquelle se dirige dans le sens inverse de la vis à recevoir (80).

27. Récipient (100) selon la revendication 26, **caractérisé en ce que** deux côtés (50b, 50d) qui sont en particulier tournés respectivement contre une dent (44, 46) sont bordés ou striés.

28. Récipient (100) selon l'une des revendications 26 ou 27, **caractérisé en ce qu'**un côté (50a) présente un bouchon (52) et qu'un autre côté (50c) présente une ouverture (54), sachant que le bouchon (52) et l'ouverture (54) coopèrent dans un système d'insertion.

29. Récipient (100) selon l'une des revendications précédentes, **caractérisé en ce que** pour chaque position de réception individuelle pour recevoir un seul matériel auxiliaire (80) dans un récipient dans lequel une pluralité de matériel auxiliaire peut être reçue, un couvercle (48) amovible propre est prévu, sachant que les différents couvercles sont reliés les uns aux autres de marnière séparable.
